(19) Europäisches Patentamt
European Patent Office
Office européen des brevets

(11) Veröffentlichungsnummer: **0 003 106**
**B1**

(12) # EUROPÄISCHE PATENTSCHRIFT

(21) Anmeldenummer: **79100018.5**

(22) Anmeldetag: **04.01.79**

(51) Int. Cl.³: **C 07 D 307/68,**
C 07 D 307/46

(54) **Verfahren zur Herstellung von substituierten Furanen**

(30) Priorität: **05.07.78 DE 2800505**

(43) Veröffentlichungstag der Anmeldung:
**25.07.79 Patentblatt 79/15**

(45) Bekanntmachung des Hinweises auf die Patenterteilung:
**14.01.81 Patentblatt 81/02**

(84) Benannte Vertragsstaaten:
**CH DE GB NL SE**

(56) Entgegenhaltungen:
**BULLETIN DE LA SOCIETE CHIMIQUE DE FRANCE,
Nr. 11, November 1971, Paris, FR,
E. BISAGNI et al.L "Furannes et pyrroles disubstituès en 2,3; XI Réaction du chloracétaldéhyde sur les β-cétoesters: formation des furannes et pyrroles attendus et de diacyl-1,4 cyclohexadiènes-1,4" Seiten 4041 - 4047
BULLETIN DE LA SOCIETE CHIMIQUE DE FRANCE,
Nr. 8, August 1967, Paris, FR
E. BISAGNI et al.: "Furannes et pyrroles disubstitués en 2,3. I,- Sur l'extension de la réaction de Feist et Bénary aux β-dicétones: nouvelle méthode de synthèse de certains furannes et pyrolles acylés en position 3", Seiten 2796 - 2801
COLLECTION CZECHOSLOVAK CHEMICAL COMMUNICATIONS, Band 31, Nr. 6 Juni 1966, Prag,
M. VALENTA et al.: "Versuche in der Furan-Reihe VI. Alkoxylierung von 2-Methyl-3-formylfuran", Seiten 2410 - 2415**

(73) Patentinhaber: **Consortium für elektrochemische Industrie GmbH
Wacker Haus Prinzregentenstrasse 22
D - 8000 München 22 (DE)**

(72) Erfinder: **Kaufmann, Rudolf, Dr.
Wackerstrasse 5
D - 8263 Burghausen (DE)
Bräunling, Hermann, Dr.
Dreyerstrasse 6
D - 8000 München 21 (DE)
Häberle, Norman, Dr.
Willibaldstrasse 51 a
D - 8000 München 21 (DE)
Müller, Reinhard, Dr.
Aretinstrasse 25
D - 8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 003 106 B1**

Verfahren zur Herstellung von substituierten Furanen

Umsetzungen zwischen β-Dicarbonylverbindungen und α-Halogencarbonylverbindungen sind unter der Bezeichung Feist-Benary-Synthese bekannt. (R. Elderfield, Th. Dodd in "Heterocyclic Compounds", Vol. 1, Editor: R. Elderfield, J. Wiley, New York, 1950, Seite 132 ff). In Gegenwart von Ammoniak werden Gemische aus Furan- und Pyrrolderivaten erhalten. Scott und Johnson, J.A.C.S., *54* 2549 (1932) erhalten ein pyrrolfreies Produkt in Ausbeuten von 50 bis 60% in Gegenwart von äquimolaren Mengen Pyridin.

Auch in Gegenwart einer Reihe anderer basischer Stoffe wie Soda, Natriumacetat, Triäthylamin, Natriumhydroxyd, Kaliumhydroxyd auch in Gegenwart von Eis (E. Bisagni et al, Bull. Soc. Chim. France 1971 (11), 4041 ff) werden nur Ausbeuten von 51%, bezogen auf β-Dicarbonylverbindungen, erreicht. In der DE-OS 20 06 472 sind sogar 3 Mol Pyridin, bezogen auf das eingesetzte Diketon, erforderlich. Angaben über die Ausbeute werden in dieser Literaturstelle jedoch nicht gemacht.

Weiterhin beschreiben Valenta et al in Collection Czechoslov. Che. Commun., Band 31, Nr. 6 (1966), Seiten 2413 und 2414, ein, gegenüber H. Winberg et al· J. Am. Chem. Soc., *82*, 1433 (1960), weiterentwickeltes Verfahren zur Herstellung von 2-Methyl-3-Ethoxy-Carbonyl-Furan. Winberg et al setzen eine 40 bis 45%-ige wäßrige Lösung von Chloracetaldehyd als eine der Reaktionskomponenten in Gegenwart eines dreimolaren Überschusses an Pyridin als Reaktionsmedium ein und erzielen 63% Ausbeute an Rohprodukt. Valenta et al setzen demgegenüber reinen (wasserfreien) Chloracetaldehyd ein und erzielen 81% Ausbeute an entsprechendem Furan. Beide Autoren verwenden mithin große Mengen an überschüssigem Pyridin. Eine Herstellungsmethode, nach der solch große Mengen an Reaktionsmedium zu verwenden sind, ist jedoch aufgrund der Notwendigkeit, große Voluminas aufzuarbeiten, schon allein aus Kostengründen und den unbefriedigenden Raum/Zeit-Leistungen der Anlagen kaum für industrielle Zwecke anwendbar.

Aufgabe der Erfindung war es somit, ein Verfahren zur Herstellung substituierter Furane zu finden, das bessere Ausbeuten liefert und den Einsatz größerer Mengen Pyridin nicht erforderlich macht.

Gegenstand der Erfindung ist ein Verfahren zur Herstellung substituierter Furane der allgemeinen Formel

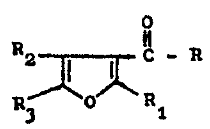

worin

R eine Alkoxygruppe mit 1 bis 6 Kohlenstoffatomen oder eine substituierte bzw. eine nichtsubstituierte Aryloxygruppe ist, vorzugsweise eine Methoxy- oder Äthoxygruppe ist, oder eine Alkylgruppe ist, vorzugsweise eine Methylgruppe,

$R_1$ eine Alkylgruppe mit 1 bis 6 Kohlenstoffatomen oder eine Arylgruppe ist, vorzugsweise eine Methylgruppe

$R_2$ und-oder $R_3$ eine Alkylgruppe mit 1 bis 6 Kohlenstoffatomen ist oder Wasserstoff, vorzugsweise Wasserstoff, ist

durch Reaktion einer β-Dicarbonylverbindung mit einer α-Chlorcarbonylverbindung in Gegenwart einer zur Bindung der entstehenden Chlorwasserstoffsäure befähigten Verbindung, bei Temperaturen zwischen 30 und 100°C, dadurch gekennzeichnet, daß die Reaktion bei pH-Werten zwischen 2 und 7, vorzugsweise bei pH-Werten zwischen 3,5 und 6, durchgeführt wird und äquimolare Mengen Erdalkalicarbonat zugegeben werden.

Als Erdalkalicarbonat eignet sich in besonderer Weise gemahlener Dolomit mit einer Teilchengröße unter 5mm. Zur Beschleunigung der Reaktion hat es sich als vorteilhaft erwiesen, bis zu 20 Mol% einer substituierten bzw. nichtsubstituierten monoheterocyclischen Monostickstoffverbindung, bezogen auf die β-Dicarbonylverbindung, einzusetzen. Der Zusatz in Mengen von 0,5 bis 5 Mol% Pyridin, bezogen auf die ß-Dicarbonylverbindung, hat sich als wirtschatlich erwiesen.

Überraschenderweise wird durch das erfindungsgemäße Verfahren gegenüber hekömmlicher Verfahrensweise nicht nur die Aufarbeitung der Reaktionsgemische wesentlich verbessert, sondern auch erheblich bessere Ausbeuten erzielt. Es können Ausbeuten bis zu 80 Mol% des gewünschten Produktes, bezogen auf eingesetzte β-Dicarbonylverbindung, erreicht werden.

Das erfindungsgemäße Verfahren zur Herstellung von substituierten Furanen erfolgt durch Umsetzung von β-Dicarbonylverbindungen mit α-Chlorcarbonylverbindungen. Als β-Dicarbonylverbindungen können β-Carbonylester, wie beispielsweise Acetessigsäuremethyl-, -äthyl-, -propyl-, -phenyl-ester, α-Chloracetessigester oder β-Diketone, wie z.B. Acetylaceton, Heptandion-3.5, 1.3-Diphenylpropandion-1.3 eingesetzt werden. Als α-Chlorcarbonyle eignen sich beispielsweise α-Chloraldehyde, wie α-Chloracetaldehyd, α-Chlorpropionaldehyd, α-Chlorbutyraldehyd oder auch α-Chlorketone, wie z.B. α-Chloraceton, 2-Chlorbutanon-3.

Als substituierte bzw. nichtsubstituierte monoheterocyclische aromatische Monostickstoffverbindung kommt vor allem Pyridin in Frage. Neben Pyridin können jedoch auch andere, wie z.B. Picoline, Lutidine oder auch Nicotinsäure zur Anwendung kommen.

Das erfindungsgemäße Verfahren zur Herstellung substituierter Furane erfolgt durch Umsetzung von β-Dicarbonylverbindungen mit α-Chlorcarbonylverbindungen. Die β-Dicarbonylverbindungen können als solche, bzw. gelöst in neutralen organischen Lösungsmitteln, eingesetzt werden.

Für das erfindungsgemäße Verfahren ist jedoch kein Lösungsmittel erforderlich. Die α-Chlorcarbonylverbindung kann ebenfalls als solche, bzw. in Form der wäßrigen, vorzugsweise der konzentrierten wäßrigen Lösung eingesetzt. Die Reaktionskomponenten werden auf die erforderliche Reaktionstemperatur zwischen 30 und 100°C, vorzugsweise 60 bis 80°C, gebracht. Als Basen werden erfindungsgemäß Erdalkalicarbonate eingesetzt. Die Teilchengröße spielt dabei keine besondere Rolle, gemahlenes Erdalkalicarbonat mit einer Teilchengröße unter 5mm hat sich als vorteilhaft erwiesen. Teilchengrößen zwischen 1 und 200 μm, insbesondere 55 μm, zeigen besonders günstiges Reaktionsverhalten. Das Erdalkalicarbonat wird in stöchiometrisch erforderlichen Mengen zugesetzt.

Beispiele für Erdalkalicarbonate sind beispielsweise Kalziumcarbonat, sowhol in gefällter als auch in natürlich vorkommender Form, wie z.B. Kalkstein, Kreide. Auch Magnesiumcarbonat, beispielsweise in form von Dolomit, eignet sich be-sonders gut. Zur Reaktionsbeschleunigung ist es zweckmäßig, substituierte bzw. nichtsubstituierte monoheterocyclische aromatische Monostickstoffverbindungen in Mengen bis zu 20 Mol% einzusetzen. Vorzugsweise eignet sich Pyridin in Mengen zwischen 0,5 bis 5 Mol%, bezogen auf die eingesetzte β-Dicarbonylverbindung, als Katalysator.

Der Vorteil des erfindungsgemäßen Verfahrens liegt u.a. auch darin, daß auf zusätzliche organische Lösungsmittel und den Einsatz wäßriger Lösungen von Basen verzeichtet werden kann. Darause ergibt sich eine nicht unerhebliche Verringerung des Reaktionsvolumens, was einen weiteren Fortschritt gegenüber dem Stand der Technik bedeutet. Die aus den Erdalkalicarbonaten im Verlauf der Reaktion sich bildenden Erdalkalihalogenide sind gut wasserlöslich und können mit der wäßrigen Phase nach der Reaktion leicht abgetrennt und/oder ausgewaschen werden.

Die Gesamtreaktion verläuft im wesentlichen in zwei Schritten, einer Kondensationsreaktion und einer Ringschlußreaktion. Das pH-Optimum der Kondensationsreaktion liegt bei pH 2 bis 7, insbesondere zwischen pH 3,5 bis 6. Die Beendigung der Kondensationsreaktion ist an der Beendigung der Kohlendioxidentwicklung zu erkennen. Danach kann die Reaktion entsprechend der eingesetzten α-Chlorcarbonylverbindung α-Chloraldehyd bzw. α-Chlorketon spezifisch auf die jeweiligen α-Chlorhalogencarbonyle abgestimmt verfahren werden. Bei der Umsetzung von α-Chloraldehyden mit β-Dicarbonylverbindungen kann die Ringschlußreaktion durch weitere Senkung des pH-Wertes verbessert werden, wobei bei der Umsetzung von α-Chlorketonen zweckmäßigerweise keine pH-Änderung gegenüber dem Kondensationsreaktionsschritt unternommen wird. Die Ringschulußreaktion schließt sich im Eintopfverfahren an die Kondensationsreaktion bei gleicher Temperatur an.

Nach Beendigung der Reaktion und Abtrennen der wäßigen Phase wird in üblicher Weise, wie z.B. durch Vakuumdestillation, aufgearbeitet.

### Beispiel 1
### Herstellung von 2-Methylfuran-3-carbonsäuremethylester

In einem Rührkessel werden 58,055 kg (500 Mol) Acetessigsäuremethylester vorgelegt und 22,5 kg (487,5 Val) gemahlener Dolomit mit einer Korngröße um 30 um eingerüht. Nach Zugabe von 0,988 kg (12,5 Mol = 2,5 Mol%) Pyridin wird auf 70°C erwärmt und dann 87,2 kg (500 Mol) 45%-ige wäßrige Chloracetaldehydlösung innerhalb etwa 1 Stunde zudosiert. Durch leichtes Kühlen wird die Reaktion bei 70° gehalten. Etwa 4 Stunden nach Reaktionsbeginn muß schwach geheizt werden, nach 6 bis 7 Stunden wird auf 75 bis 80° geheizt, die Heizung abgestellt und noch einige Zeit bei fallender Temperatur weitergerührt. Das auf Raumtemperatur abgekühlte Reaktionsgemisch wird portionsweise mit conc. Salzsäure versetzt, um überschüssigen Dolomit zu zersetzen. Nach Phasentrennung wird die wäßrige Unterschicht abgetrennt, die organische Schicht mit 76 l Wasser gewaschen. Zur besseren Phasentrennung können ca. 2 kg Kochsalz zugegeben werden. Die nun unten befindliche organische Phase wird abgetrennt und bei vermindertem Druck destilliert. Die Ausbeute an 2-Methylfuran-3-carbonsäuremethylester beträgt 53,6 kg = 76,6% der Theorie.

### Beispiel 2
### Herstellung von 2-Methyl-3-acetylfuran

100,12g Acetylaceton werden entsprechend Beispiel 1 mit 44 g Dolomitmehl und 4 ml (5 Mol%) Pyridin, dann innerhalb 0,5 Stunden bei 70° mit 174,4 g 45%-iger wäßriger Chloracetaldehydlösung versetzt. Es wird bei 70° 4,5 Stunden, dann noch 1 Stunde bei 75° gerührt. Das entstehende $CO_2$-Volumen bei Raumtemperatur beträgt etwa 10,8 1. Aufarbeitung erfolgt wie in Beispiel 1. Die Fraktion von 59 bis 61° bei 11 Torr wird gesammelt; sie beträgt 93,56 g entsprechend 75,5% der Theorie.

NMR-Spektrum ($CDCl_3$, TMS als int. Stand):

2,38,S,3H; 2,54,S,3H;

6,75,d,J = 2,1 Hz,1H;

7,38,d,J = 2,1 Hz,1H

**Patentansprüche**

1. Verfahren zur Herstellung substituierter Furane der allgemeinen Formel

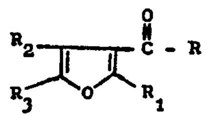

worin

R eine Alkoxygruppe mit 1 bis 6 Kohlenstoffatomen oder eine substituierte bzw. nichtsubstituierte Aryloxygruppe, vorzugsweise eine Methoxy- oder Athoxygruppe ist, oder eine Alkylgruppe, vorzugsweise eine Methylgruppe ist,

$R_1$ eine Alkylgruppe mit 1 bis 6 Kohlenstoffatomen, oder eine Aryl-Grüppe, vorzugsweise eine Methylgruppe ist,

$R_2$ und/oder $R_3$ eine Alkylgruppe mit 1 bis 6 Kohlenstoffatomen oder Wasserstoff, vorzugsweise Wasserstoff, ist

durch Reaktion einer $\beta$-Dicarbonylverbindung mit einer $\alpha$-Chlorcarbonylverbindung in Gegenwart einer zur Bindung der entstehenden Chlorwasserstoffsäure befähigten Verbindung, bei Temperaturen zwischen 30 und 100°C, dadurch gekennzeichnet, daß die Reaktion bei pH-Werten zwischen 2 und 7, vorzugsweise bei pH-Werten zwischen 3,5 und 6 durchgeführt wird und äquimolare Mengen Erdalkalicarbonat zugegeben werden.

2. Verfahren nach Anspruch 2, dadurch gekennzeichnet, daß zusätzlich 0,1 bis 20 Mol% einer substituierten bzw. nichtsubstituierten monoheterocyclischen aromatischen Monostickstoffverbindung, bezogen auf die $\beta$-Dicarbonylverbindung, zugesetzt wird.

3. Verfahren nach Anspruch 1, dadurch gekennzeichnet, daß zusätzlich 0,5 bis 5 Mol% Pyridin, bezogen auf die $\beta$-Dicarbonylverbindung, zugesetzt wird.

4. Verfahren nach Anspruch 1 bis 3, dadurch gekennzeichnet, daß als Erdalkalicarbonat gemahlener Dolomit mit einer Teilchengröße unter 5mm zugesetzt wird.

**Revendications**

1. Procédé de préparation de furannes substitués répondant à la formule générale

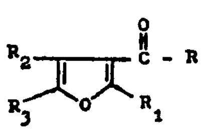

dans laquelle

R représente un radical alcoxy contenant de 1 à 6 atomes de carbone ou un radical aryloxy substitué ou non, de préférence un radical méthoxy ou éthoxy, ou encore un radical alkyle, de préférence un radical méthyle,

$R_1$ représente un radical alkyle contenant de 1 à 6 atomes de carbone ou un radical aryle, de préfefence un radical méthyle,

$R_2$ et/ou $R_3$ représente un radical alkyle contenant de 1 à 6 atomes de carbone ou un atome d'hydrogène, de préférence un atome d'hydrogène,

par réaction d'un composé $\beta$-dicarbonylique avec un composé $\alpha$-chlorocarbonylique en présence d'un composé capable de fixer l'acide chlorhydrique formé, à des températures comprises entre 30 et 100°C, procédé caractérisé en ce qu'on effectue la réaction à des pH compris entre 2 et 7, de préférence entre 3,5 et 6, et on ajoute des quantités équimolaires d'un carbonate de métal alcalinoterreux.

2. Procédé selon la revendication 1, caractérisé en ce qu'on ajoute en outre de 0,1 à 20% en moles d'un composé monoazoté aromatique monohétérocyclique, substitué ou non, par rapport au composé $\beta$-dicarbonylique.

3. Procédé selon la revendication 1, caractérisé en ce qu'on ajoute en outre de 0,5 à 5% en moles de pyridine, par rapport au composé $\beta$-dicarbonylique.

4. Procédé selon l'une quelconque des revendications 1 à 3, caractérisé en ce qu'on ajoute, comme carbonate de métal alcalinoterreux, de la dolomite broyée d'une granularité inférieure à 5mm.

**Claims**

1. Process for the manufacture of substituted furans of the general formula

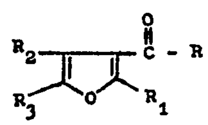

in which

R is an alkoxy group having 1 to 6 carbon atoms or a substituted or unsubstituted aryloxy group, preferably a methoxy or ethoxy group, or an alkyl group, preferably a methyl group,

$R_1$ is an alkyl group having 1 to 6 carbon atoms or an aryl group, preferably a methyl group, and

$R_2$ and/or $R_3$ is an alkyl group having 1 to 6 carbon atoms or hydrogen, preferably hydrogen,

by reacting a $\beta$-dicarbonyl compound with an $\alpha$-chlorocarbonyl compound in the presence of a compound that is capable of binding the resulting hydrochloric acid, at temperatures of between 30 and 100°C, *characterised in that* the reaction is carried out at pH values of between 2 and 7, preferably at pH values of between 3.5 and 6, and that equimolar quantities of alkaline earth metal carbonate are added.

2. Process according to claim 1, *characterised in that* from 0.1 to 20 mole%, based on the $\beta$-dicarbonyl compound, of a substituted or unsubstituted monoheterocyclic aromatic mononitrogen compound are additionally added.

3. Process according to claim 1, *characterised in that* from 0.5 to 5 mole%, based on the $\beta$-dicarbonyl compound, of pyridine are additionally added.

4. Process according to claims 1 to 3, *characterised in that* ground dolomite having a particle size of less than 5mm is added as the alkaline earth metal carbonate.